Europäisches Patentamt

European Patent Office

Office européen des brevets

(19)

(11) Veröffentlichungsnummer: **0 259 668 B1**

(12) # EUROPÄISCHE PATENTSCHRIFT

(45) Veröffentlichungstag der Patentschrift:
12.12.90

(21) Anmeldenummer: 87112040.8

(22) Anmeldetag: 19.08.87

(51) Int. Cl.⁵: **A61M 5/14**, F04B 21/02

(54) Kolbenpumpe für ein Medikamentendosiergerät.

(30) Priorität: 01.09.86 DE 3629698

(43) Veröffentlichungstag der Anmeldung:
16.03.88 Patentblatt 88/11

(45) Bekanntmachung des Hinweises auf die Patenterteilung:
12.12.90 Patentblatt 90/50

(84) Benannte Vertragsstaaten:
DE FR GB IT SE

(56) Entgegenhaltungen:
EP-A- 0 102 824
DE-A- 1 911 649
US-A- 3 974 854
US-A- 4 482 346

(73) Patentinhaber: Siemens Aktiengesellschaft,
Wittelsbacherplatz 2, D-8000 München 2(DE)

(72) Erfinder: Buchholtz, Gerhard, Dipl.-Ing., Im
Heuschlag 26, D-8520 Erlangen(DE)
Erfinder: Obermann, Peter, Dipl.-Ing., Am Färberhof 12,
D-8520 Erlangen(DE)
Erfinder: Fickweiler, Werner, Anderlohrstrasse 56,
D-8520 Erlangen(DE)

**Beschreibung**

Die Erfindung betrifft eine Kolbenpumpe für ein Medikamentendosiergerät mit einem durch eine Betätigungseinrichtung verschiebbaren Kolben, zwischen dem und einer ihn umgebenden Zylinderwand ein Spalt (31, 118), der eine Einlaßkammer (7) und eine Auslaßkammer (100) miteinander verbindet, vorgesehen ist, mit einem Fluidtrakt, der eine Einlaßleitung und die Einlaßkammer umfaßt, und mit einem in der Einlaßkammer beweglichen Ventilteil, das zum Verschießen einer Einlaßöffnung an der Einlaßleitung vorgesehen ist.

Bei einem implantierbaren Medikamentendosiergerät ist es aus Gründen der Miniaturisierung vorteilhaft, als Pumpaggregat eine miniaturisierte Kolbenpumpe einzusetzen. Der in diesem Anwendungsfall benötigte Hubraum einer solchen Pumpe liegt in der Größenordnung von 1 µl. Die Pumpe sollte in der Lage sein, auch Gasblasen mit einer zufriedenstellenden Förderrate zu pumpen, damit der Patient bei Auftreten einer Gasblase im Fluidtrakt nicht längere Zeit das Medikament entbehren muß, d.h. unversorgt bleibt. Eine Gasblase im Medikamentenspeicher entsteht beispielsweise durch Ausgasen des Medikaments oder beim Nachfüllen von Medikamenten. Das Problem der Gasblasenförderung stellt sich noch verstärkt bei Medikamentendosiergeräten, bei denen der Medikamentenspeicher mit einem Unterdruck beaufschlagt ist. Ein solcher aus sicherheitstechnischen Gründen vorteilhafter Unterdruck im Medikamentenspeicher schwankt typischerweise zwischen 0, 5 und 1 bar absolut. Dadurch ergibt sich aus physikalischen Gründen bei einer idealen Pumpe mit totraumfreier Einlaßkammer eine Reduzierung der Gasförderrate an der unteren Grenze des Unterdrucks (0, 5 bar absolut) um den Faktor 2 gegenüber der Flüssigkeitsförderrate. Hat eine Pumpe einen Totraum in der Einlaßkammer, der die gleiche Größe wie der Hubraum aufweist, ist aus physikalischen Gründen an der unteren Grenze des Unterdrucks (0,5 bar absolut) im speichersystem eine Förderung von Gasblase unmöglich. Eine in der Einlaßkammer befindliche Gasblase wird lediglich komprimiert und dekomprimiert, ohne daß ein Fördervorgang zur Auslaßseite hin stattfindet. Aus den genannten Gründen ist es anzustreben, bei einer Kolbenpumpe ein möglichst großes Verhältnis von Hubraum zu Totraum in der Einlaßkammer zu erreichen. Da bei einem Medikamentendosiergerät kleine Flüssigkeitsmengen zu fördern sind, ist eine Vergrößerung des Hubraums der Kolbenpumpe in der Regel nicht möglich. Der Ansatzpunkt für die Erzielung einer großen Gasförderrate liegt also in der Reduzierung des Totraums.

Eine Kolbenpumpe der eingangs genannten Art ist aus der; EP-A 0 102 824 bekannt. Dort ist eine Einlaßkammer vorgesehen, die von der Stirnfläche eines Kolbens, einer zylindrischen Wand und einer Gehäusestirnfläche mit einer Öffnung für eine Einlaßleitung gebildet wird. Innerhalb der Einlaßkammer ist ein bewegliches Ventilteil angeordnet. Eine Spiralfeder hält das Ventilteil in einer Ruhestellung gegen die Einlaßöffnung gedrückt. Durch die Spiralfeder mit zugehörigen Befestigungsmitteln entsteht ein relativ großer Totraum, der sich unter Umständen ungünstig auf die Gasförderrate der Kolbenpumpe auswirken kann.

Aus der DE-A 1 911 649, Fig. 1 bis 4, ist ein Überströmventil bekannt, das vorzugsweise in Blutkreisläufen als Sicherheits- und Regelventil werden kann. Das Ventil weist ein mit von einstellbarer magnetischer Schließkraft auf. Jedoch sind hier das Ventilteil und der Ventilsitz strömungsgünstig ausgebildet. Das bedeutet, daß keine scharfen Übergänge oder vorspringende Halterungen vorhanden sind, was zu erhebliches Totraum führt. Außerdem ist nach Fig. 2 und 4 bei geschlossenem Ventil ein Leckfluß vorhanden, um eine Gerinselbildung zu verhindern. Auch diese Konstruktion bedeutet das Vorhandensein erheblicher Toträume.

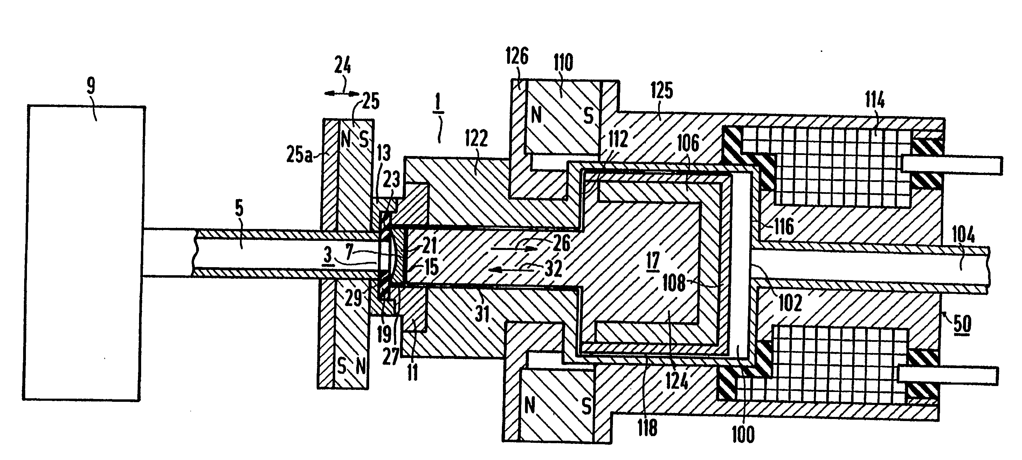

Aufgabe der Erfindung ist es, eine Kolbenpumpe der eingangs genannten Art anzugeben, die eingangsseitig einen kleinen Totraum aufweist und somit eine hohe Gasförderrate ermöglicht.

Diese Aufgabe wird erfindungsgemäß dadurch gelöst, daß das Ventilteil in der Einlaßkammer frei bewegbar untergebracht ist, daß die Stirnfläche des Kolbens eine Wand der Einlaßkammer bildet, daß ein Spalt zwischen dem Kolben und der umgebenden Zylinderwand vorgesehen ist, und daß außerhalb des Einlaß-Fluidtrakts ein Bauteil angeordnet ist, das mit dem Ventilteil in magnetischer Wechselwirkung steht und mit diesem ein magnetisches Federsystem bildet, welches auf das Ventilteil eine Schließkraft zum Verschließen der Einlaßöffnung ausübt.

Eine besonders vorteilhafte Ausführungsform der Kolbenpumpe zeichnet sich dadurch aus, daß das Ventilteil teilweise oder vollständig aus einem magnetisierbaren Material besteht und daß das Bauteil ein Dauermagnet ist.

Bei der erfindungsgemäßen Kolbenpumpe wird in der Einlaßkammer ein sehr kleiner Totraum erzielt; dementsprechend ist das Verhältnis von Hubraum zu Totraum der Einlaßkammer groß, wodurch eine hohe Gasförderrate möglich ist.

Als weiterer Vorteil ergibt sich, daß die Kraft auf das Ventilteil aufgrund der die Pumpkammerwände durchdringenden Magnetwirkung noch nachträglich von außen einstellbar ist, d.h. nachdem die Kolbenpumpe bereits zusammengebaut ist. Die Zuverlässigkeit wird durch den Einsatz des magnetischen Federsystems gewährleistet. Die mit einer mechanischen Feder einhergehende Materialermüdung und/oder Korrosion treten nicht auf. Desweiteren ist es möglich, das in der Einlaßkammer befindliche Ventilteil einfach und flächig zu gestalten, was sich günstig beim Pumpen von Medikamenten mit Sedimentationsneigung, wie z.B. Insulin, auswirkt. Außerdem entfallen durch die Anordnung des genannten Bauteils außerhalb der Einlaßkammer Probleme, die durch eine Wechselwirkung des Medikaments mit dem Werkstoff einer innen angeordneten mechanischen Feder entstehen könnten. Einerseits braucht also nicht auf die Medikamentenkompatibilität des Federwerkstoffs geachtet zu werden, und andererseits besteht keine Gefahr einer durch den Federwerkstoff hervorgerufenen Toxizität des Medikaments, z.B. durch auslösbare Nickelbestandteile im Federwerkstoff.

Weitere Vorteile und Ausgestaltungen der Erfindung ergeben sich aus der Beschreibung eines Ausführungsbeispiels anhand der Figur.

Die Figur zeigt die schematische Darstellung einer Kolbenpumpe für die Medikamentendosierung mit einem magnetischen Federsystem an der Einlaßkammer.

Nach der Figur umfaßt eine Kolbenpumpe 1 eingangsseitig einen Fluidtrakt 3, der eine Einlaßleitung 5 und eine Einlaßkammer 7 besitzt. Die eine Seite der Einlaßleitung 5 ist an ein Medikamentenreservoir 9 angeschlossen, das vorzugsweise mit einem Unterdruck beaufschlagt ist. Die andere Seite der Einlaßleitung 5 mündet in die Einlaßkammer 7.

Die Einlaßkammer 7 ist begrenzt durch ein zylindrisches Mantelteil 11, der Stirnfläche 15 eines Kolbens 17 und der Zylindermantelfläche sowie der ebenen Fläche eines Ventilteils 21. Ein Flansch 13 schließt sich an das Mantelteil 11 an und weist eine zentrale Öffnung auf, an deren Rand die Einlaßleitung 5 durch Kleben oder Schweißen befestigt ist. Alternativ hierzu ist es möglich, den Flansch 13 und die Einlaßleitung 5 aus einem Teil herzustellen. Das Mantelteil 11 weist innen einen Durchmesser von ca. 2 mm auf.

An dem Flansch 13 liegt eine ringförmige Ventildichtung 19 an, die vonh dem zylindrischen Mantelteil 11 gegen den Flansch 13 gedrückt wird. Die Ventildichtung 19 weist eine zentrale Öffnung auf, die dem inneren Querschnitt der Einlaßleitung 5 entspricht. Innerhalb der Einlaßkammer 7 ist das Ventilteil 21 beweglich angeordnet. Das Ventilteil 21 ist so geformt, daß es bei Anliegen gegen die Ventildichtung 19 einen flüssigkeits- und gasdichten Verschluß bildet. Im dargestellten Fall ist das Ventilteil 21 an seiner dichtenden Seite derart ausgehölt, d.h. nach innen gewölbt, daß randseitig eine Schneidekante 23 vorhanden ist, welche im Ruhezustand der Kolbenpumpe 1 federnd gegen die Ventildichtung 19 gedrückt ist. Auf der anderen Seite besitzt das Ventilteil 21 eine ebene Stirnfläche.

Der im Ruhezustand dichtende Ventilsitz des Ventilteils 21, d.h. dessen Anliegen gegen die Ventildichtung 19, wird durch ein Bauteil 25, hier ein Dauermagnetsystem, erreicht. Dieses Bauteil 25 besteht hier z.B. aus zwei würfel- oder blockförmigen Permanentmagneten, die auf einem magnetischen Rückschluß 25a aus einem magnetisierbaren Material einander gegenüberliegend befestigt sind. Die Nord- und Südpole sind mit N bzw. S bezeichnet. Das Bauteil 25 sitzt fest auf der Einlaßleitung 5 und bildet zusammen mit dem Ventilteil 21 ein magnetisches Federsystem. Dabei zieht es das aus einem magnetisierbaren Material bestehende Ventilteil 21 durch magentische Feldwirkung gegen die Ventildichtung 19. Ebenso ist es möglich, das Ventilteil 21 aus einem Dauermagneten zu fertigen. In diesem Fall ist es möglich, das Bauteil 25 als Dauermagneten oder als Bauelement aus einem magnetisierbaren Werkstoff zu gestalten. Alternativ hierzu kann das Bauteil 25 in Form eines Dauermagneten auch auf der anderen, d.h. der rechten Seite, des Ventilteils 21 angeordnet sein und anstatt einer ziehenden Wirkung eine drückende Wirkung gegen die Ventildichtung 19 erzeugen. Wesentlich dabei ist, daß das Bauteil 25 (d.h. der Dauermagnet oder als Alternative der magnetisierbare Werkstoff) außerhalb des Fluidtrakts 3 angeordnet ist. Auf diese Weise entsteht innerhalb des Fluidtrakts 3, insbesondere innerhalb der Einlaßkammer 7, kein unnötiger Totraum.

Das Bauteil 25 ist zweckmäßigerweise entlang der Symmetrie- oder Längsachse des Ventilteils 21 verschiebbar angeordnet. Dies ist durch einen Doppelpfeil 24 angedeutet. Das ermöglicht es, die Rückstellkraft auf das Ventilteil 21 bei fertig montierter Pumpe 1 nachträglich einzustellen.

Im Betrieb der Kolbenpumpe 1 wird der Kolben 17 z.B. durch elektromagnetische Feldwirkung, wie später näher erläutert wird, in Richtung des Pfeils 26 nach rechts bewegt, wodurch in der Einlaßkammer 7 ein starker Unterdruck entsteht. Sobald die durch den Unterdruck in der Einlaßkammer 7 auf das bewegbare Ventilteil 21 ausgeübte Kraft die Rückstellkraft des magnetischen Federsystems überschreitet, hebt sich die Schneidekante 23 des bewegbaren Ventilteils 21 von der Ventildichtung 19 ab, und ein Ventilspalt 29 zwischen dem bewegbaren Ventilteil 21 und der Ventildichtung 19 wird geöffnet. Das Medikament wird gegen den Unterdruck im Medikamentenreservoir 9 durch den starken Unterdruck in der Einlaßkammer 7 in diese hineingesaugt. Dabei durchströmt es die Einlaßleitung 5, den geöffneten Ventilspalt 29 zwischen dem bewegbaren Ventilteil 21 und der Ventildichtung 19 und einen Spalt 27 zwischen dem Rand des bewegbaren Ventilteils 21 und dem zylindrischen Mantelteil 11. Hat der Kolben 17 seine Hubstellung erreicht, verringert sich durch das einströmende Medikament der starke Unterdruck in der Einlaßkammer 7. Sobald die durch den Unterdruck in der Einlaßkammer 7 auf das bewegbare Ventilteil 21 ausgeübte Kraft die Rückstellkraft des Federsystems unterschreitet, senkt sich das Ventilteil 21 wieder vollständig auf die Ventildichtung 19, und der Ventilspalt 29 wird geschlossen. Flüssiges Medikament befindet sich jetzt zwischen der Stirnfläche 15 des Kolbens 17 und der ebenen Stirnfläche des Ventilteils 21. Wird der Kolben 17 von seiner Hubstellung in Richtung des Pfeils 32 in seine (gezeigte) Senkstellung bewegt, so wird auf das in der Einlaßkammer 7 befindliche Medikament ein Druck ausgeübt. Hierdurch wird das Medikament durch einen Ringspalt 31 zwischen dem Kolben 17 und dessen Führungsgehäuse 122 in Richtung auf eine Auslaßleitung 104 am Auslaßsystem 50 gedrückt. Der Spalt 31 kann auch in Form eines oder mehrerer Längskanäle ausgebildet sein.

Wie bereits erwähnt, wird der Kolben 17 ebenfalls durch magnetische Feldwirkung in seine Ruhestellung, also seine Senkstellung, geführt. Die dazu notwendigen Mittel werden im folgenden beschrieben.

Das Auslaßsystem 50 umfaßt eine Pumpkammer (Auslaßkammer) 100, die eine Auslaßöffnung 102 aufweist. Von der Auslaßöffnung 102 führt eine Auslaßleitung 104 zu einem Katheteranschluß (nicht gezeigt). Die Pumpkammer 100 wird gebildet durch ihre beiden Stirnseiten 112 und 116, durch ein weiteres und größeres zylindrisches Mantelteil 122, durch die Auslaßöffnung 102, durch den im Auslaßbereich erweiterten Kolben 17 und durch einen Spalt 118. Ein mit dem Kolben 17 verbundenes Ankerteil 106, das von einer topfförmigen Kapselung 108 eingefaßt ist, befindet sich innerhalb der Pumpkammer 100. Das weitere Mantelteil 122 und das Mantelteil 11 können auch aus einem einzigen Stück bestehen.

Im gezeigten Ausführungsbeispiel ist das Ankerteil 106 topfförmig ausgebildet und über den Kern eines am Kolben 17 angebrachten Ankerträgers 124 gestülpt. Das Ankerteil 106 ist aus einem magnetisierbaren Material, beispielsweise Weicheisen, gefertigt. Um eine Wechselwirkung mit dem flüssigen Medikament zu vermeiden, ist die Kapselung 108 so um das Ankerteil 106 gelegt und mit dem am Kolben 17 angebrachten Ankerträger 124 verbunden, daß eine vollständige Kapselung des Ankerteils 106 erzielt wird.

Ein Permanentmagnet 110 ist außerhalb der Pumpkammer 100 angebracht. Der Permanentmagnet 110 ist beispielsweise ein Ringmagnet, der so am äußeren Gehäuse 125 der Kolbenpumpe 1 plaziert ist, daß auf das Ankerteil 106 permanent eine Kraft wirkt, die es von der Auslaßöffnung 102 wegzieht. Als Anschlag für diese Wegziehbewegung kann die Stirnseite 112 der Pumpkammer 100 dienen; bevorzugt liegt der Kolben 17 jedoch zur Vermeidung von Totraum in der Einlaßkammer 7 mit seiner Stirnfläche 15 am Ventilteil 21 an. Befindet sich der Ankerträger 124 an der Stirnseite 112, so ist der Kolben 17 in einer Ruhelage, d.h. in unbetätigtem Zustand. Der Permanentmagnet 110 ist vorzugsweise in Richtung der Kolbenlängsachse verschiebbar (nicht gezeigt), wodurch die Rückstellkraft auf den Kolben 17 kontinuierlich einstellbar ist. Der Permanentmagnet 110 ist zwischen dem zylindrischen Gehäuse 125 und einem magnetischen Rückschlußteil 126 gehalten.

Der Ankerteil 106 dient gleichzeitig als Anker für ein im wesentlichen aus einer elektromagnetischen Spule 114 bestehendes elektromagnetisches Antriebssystem, das an bestimmter Stelle der Kolbenpumpe 1 angeordnet ist. Bei der Wahl der Stelle ist lediglich darauf zu achten, daß das beim Erregen der elektromagnetischen Spule 114 mit einem Stromimpuls erzeugte magnetische Feld eine ausreichende Kraftwirkung auf das Ankerteil 106 hat (das dann als Anker für die Spule 114 anzusehen ist), so daß der Kolben 17 in Richtung auf die Auslaßöffnung 102 bis zum Anschlag der Kapselung 108 an die andere Stirnseite 116 der Pumpkammer 100 geführt wird.

Die Funktionen des Permanentmagneten 110 und des Ankerteils 106 können auch vertauscht werden. Dann würde ein Ankerteil aus einem dauermagnetischen Stoff und anstelle des Permanentmagneten ein Weicheisenring eingesetzt werden. Als äußerer Bestandteil des magnetischen Federsystems ist dann nicht der Permanentmagnet 110 anzusehen, sondern der Weicheisenring. Es ist weiterhin denkbar, daß das Federsystem aus dem außerhalb der Pumpkammer 100 befindlichen Permanentmagneten 110 und einem Ankerteil 106 aus einem dauermagnetischen Werkstoff besteht. Alternativ hierzu kann der Permanentmagnet 110 auch auf der anderen Seite des Ankerteils 106 angeordnet sein, also auf der Seite, die der Auslaßöffnung 102 zugewandt ist; anstelle einer ziehenden Kraft würde dann eine drückende Kraft auf das Ankerteil 106 einwirken, die es von der Auslaßöffnung 102 wegdrückt.

Beim Fließen eines Stroms in der Spule 114 wird der Kolben 17 also in Richtung der Auslaßöffnung 102 bewegt. Er drückt dabei das flüssige Medikament einerseits durch die Auslaßleitung 104 und andererseits über den Spalt 118, der grundsätzlich breiter ist als der Ringspalt 31, in die Kammer, die zwischen der Stirnwand 112 und dem Kolben 17 entsteht. Wird der Stromfluß durch die Spule 114 unterbrochen, so wird das von der Kapselung 108 und dem Ankerträger 124 umschlossene Ankerteil 106 durch den Permanentmagneten 110 zur ersten Stirnseite 112 zurückgeführt, wobei auch der mit dem Ankerträger 124 verbundene Kolben 17 in seine Ruhestellung zurückgeführt wird. Während dieser Zurückführungsbewegung wird das flüssige Medikament, welches beim Hub des Kolbens 17 aus dem Reservoir 9 in die Einlaßkammer 7 innerhalb des Fluidtraktes 3 geflossen ist, durch die Spalte 31 und 118 zur Pumpkammer 100 gefördert. Der Ringspalt 31 erstreckt sich dabei zwischen einem Teil des Führungsgehäuses 122 und dem dünneren Teil des Kolbens 17. Ist der Kolben 17 in seine Ruhelage zurückgeführt, so ist gleichzeitig die Pumpkammer 100 mit flüssigem Medikament nachgefüllt. Der Pumpvorgang wiederholt sich in gleicher Art.

Eine für den medizinischen Anwendungsfall geeignete Dimensionierung, z.B. für ein implantierbares Insulin-Dosiergerät, sieht einen Durchmesser des Kolbens 17 im Bereich des Spalts 31 von 2 mm vor. Ein einziger Kolbenhub von ca. 0,35 mm würde dann zu einer geförderten Flüssigkeitsmenge von ca. 1 µl führen.

Durch das (z.T. außerhalb des Fluidtrakts 3 liegende) Federsystem, im Ausführungsbeispiel durch das Bauteil 25 gebildet, wird in der Einlaßkammer 7 selbst ein sehr kleiner Totraum erzielt. Das Verhältnis von Hubraum zu Totraum ist hoch, wodurch bei Vorliegen einer Gasblase in der Einlaßleitung 5 diese in akzeptabler Weise weiterbefördert wird. Gleichzeitig können keine Wechselwirkungen zwischen dem Federsystem und dem Medikament auftreten. Die Gefahr einer frühzeitigen Alterung der Federwerkstoffe oder einer Vergiftung des Medikaments ist nicht gegeben.

**Patentansprüche**

1. Kolbenpumpe für ein Medikamentendosiergerät mit einem Kolben (17), der durch eine Betätigungseinrichtun verschiebbar ist, mit einem Einlaß-Fluidtrakt (3), der eine Einlaßleitung (5) und eine Einlaßkammer (7) umfaßt, und mit einem in der Einlaßkammer (7) beweglichen Ventilteil (21), das zum Verschließen einer Einlaßöffnung an der Einlaßleitung (5) vorgesehen ist, **dadurch gekennzeichnet,** daß das Ventilteil (21) in der Einlaßkammer (7) frei beweglich untergebracht ist, daß die Stirnfläche (15) des Kolbens (17) eine Wand der Einlaßkammer (7) bildet, daß ein Spalt (31) zwischen dem Kolben (17) und der umgebenden Zylinderwand vorgesehen ist, und daß außerhalb des Einlaß-Fluidtrakts (3) ein Bauteil (25) angeordnet ist, das mit dem Ventilteil (21) in magnetischer Wechselwirkung steht und mit diesem ein magnetisches Federsystem bildet, welches auf das Ventilteil (21) eine Schließkraft zum Verschließen der Einlaßöffnung ausübt.

2. Kolbenpumpe nach Anspruch 1, **dadurch gekennzeichnet,** daß das Ventilteil (21) einen magnetisierbaren Werkstoff enthält, und daß das Bauteil (25) ein Dauermagnet ist.

3. Kolbenpumpe nach Anspruch 1, **dadurch gekennzeichnet,** daß das Ventilteil (21) einen Dauermagneten enthält, und daß das Bauteil (25) ein Dauermagnet ist.

4. Kolbenpumpe nach Anspruch 1, **dadurch gekennzeichnet,** daß das Ventilteil (21) einen Dauermagneten enthält, und daß das Bauteil (25) einen magnetisierbaren Werkstoff umfaßt.

5. Kolbenpumpe nach einem der vorangehenden Ansprüche, **dadurch gekennzeichnet,** daß das Bauteil (25) entlang der Längsachse des Ventilteils (21) verschiebbar ist.

6. Kolbenpumpe nach einem der vorangehenden Ansprüche, **dadurch gekennzeichnet,** daß die Einlaßkammer (7) zylindrisch mit einem Durchmesser von ca. 2 mm ausgebildet ist.

7. Kolbenpumpe nach einem der vorangehenden Ansprüche, **dadurch gekennzeichnet,** daß an der dichtenden Seite des Ventilteils (21) durch Aushöhlung eine randseitige Schneidkante (23) gebildet ist, die im Ruhezustand der Kolbenpumpe (1) gegen eine Ventildichtung (19) gedrückt ist.

8. Kolbenpumpe nach einem der vorangehenden Ansprüche, **dadurch gekennzeichnet,** daß das Ventilteil (21) eine dem Kolben (17) gegenüberliegende ebene Seite aufweist.

## Claims

1. Piston pump for a dosed medicament delivery device having a piston (17) that can be displaced by an actuating device and between which and a cylinder wall that surrounds said piston a gap is provided which connects an inlet chamber (7) and an outlet chamber (100) to one another, having a fluid tract (3) that comprises an inlet line (5) and the inlet chamber (7), and having a valve part (21) that is movable in the inlet chamber (7) and is provided for closing an inlet opening on the inlet line (5), characterized in that the valve part (21) is accommodated in the inlet chamber (7) in a freely movable fashion independently of the piston (17), in that in the non-actuated state the piston (17) is located with one end face (15) located immediately adjacent to, or bearing directly upon a corresponding face of the valve part (21), and in that outside the fluid tract (3) a component (25) is arranged which together with the valve part (21) forms by means of a magnetic interaction a magnetic spring system, which exerts a closing force on the valve part (21) to close the inlet opening.

2. Piston pump according to Claim 1, characterized in that the valve part (21) contains a magnetizable material, and in that the component (25) is a permanent magnet.

3. Piston pump according to Claim 1, characterized in that the valve part (21) contains a permanent magnet, and in that the component (25) is a permanent magnet.

4. Piston pump according to Claim 1, characterized in that the valve part (21) contains a permanent magnet, and in that the component (25) comprises a magnetizable material.

5. Piston pump according to one of the preceding claims, characterized in that the component (25) can be displaced along the longitudinal axis of the valve part (21).

6. Piston pump according to one of the preceding claims, characterized in that the inlet chamber (7) is constructed in a cylindrical fashion with a diameter of approx. 2 mm.

7. Piston pump according to one of the preceding claims, characterized in that a cutting edge (23), which in the state of rest of the piston pump (1) is pressed against a valve seal (19), is formed at the rim on the sealing side of the valve part (21) by hollowing out.

8. Piston pump according to one of the preceding claims, characterized in that the valve part (21) has a flat side opposite the piston (17).

## Revendications

1. Pompe à piston pour appareil de dosage de substances médicamenteuses, comprenant un piston (17), qui peut être déplacé par un dispositif d'actionnement et entre lequel et une paroi de cylindre l'entourant est prévu un intervalle (31, 118) mettant en communication mutuelle une chambre d'admission (7) et une chambre de refoulement (100), une voie de commutation ou de passage pour le fluide (3), qui se compose d'un conduit d'admission (5) et de la chambre d'admission (7), et un obturateur (21) mobile dans la chambre d'admission (7) et destiné à fermer un orifice d'admission du conduit d'admission (5), caractérisée en ce que l'obturateur (21) est logé dans la chambre d'admission (7) avec possibilité de se déplacer librement, indépendamment du piston (17), en ce qu'une surface frontale (15) du piston (17), lorsqu'il n'est pas actionné, est au voisinage immédiat d'une surface correspondante de l'obturateur (31) ou s'y applique directement, et en ce que, à l'extérieur de la voie de passage pour le fluide (3) est prévu un élément (25) qui forme avec l'obturateur (21), par interaction magnétique, un système magnétique élastique, qui applique à l'obturateur (21) une force de fermeture pour fermer l'orifice d'admission.

2. Pompe piston suivant la revendication 1, caractérisée en ce que l'obturateur (21) comporte un matériau magnétisable, et en ce que l'élément (25) est un aimant permanent.

3. Pompe à piston suivant la revendication 1, caractérisée en ce que l'obturateur (21) comporte un aimant permanent, et en ce que l'élément (25) est un aimant permanent.

4. Pompe à piston suivant la revendication 1, caractérisée en ce que l'obturateur (31) comporte un aimant permanent, et en ce que l'élément (25) comprend un matériau magnétisable.

5. Pompe à piston suivant l'une des revendications précédentes, caractérisée en ce que l'élément (25) peut coulisser le long de l'axe longitudinal de l'obturateur (21).

6. Pompe à piston suivant l'une des revendications précédentes, caractérisée en ce que la chambre d'admission (7) est cylindrique en ayant un diamètre de 2 mm environ.

7. Pompe à piston suivant l'une des revendications précédentes, caractérisée en ce que, du côté assurant l'étanchéité de l'obturateur (21), est formée, par évidement, une arête vive (23) qui se trouve du côté marginal et qui est repoussée, à l'état de repos de la pompe a piston (1), sur une garniture d'étanchéité (19).

8. Pompe à piston suivant l'une des revendications précédentes, caractérisée en ce que l'obturateur (21) présente un côté plan opposé au piston (17).